# EUROPEAN PATENT SPECIFICATION

(11) **EP 1 326 617 B1**
(45) Date of publication and mention of the grant of the patent: **02.08.2006**
(21) Application number: 01987669.7
(22) Date of filing: 17.10.2001
(51) Int. Cl.: A61K 31/57

(54) **USE OF 11BETA-(4-ACETYLPHENYL)-17BETA-HYDROXY-17ALPHA-(1,1,2,2-PENTAFLUOROETHYL)ESTRA-4,9-DIEN-3-ONE FOR THE PREPARATION OF A MEDICAMENT FOR THE TREATMENT FO BREAST, OVARIAN, ENDOMETRIAL CANCER, MYELOMA AND MENINGIOMA**
VERWENDUNG VON 11BETA-(4-ACETYLPHENYL)-17BETA-HYDROXY-17ALPHA-(1,1,2,2-PENTAFLUOROETHYL)ESTRA-4,9-DIEN-3-ON ZUR HERSTELLUNG EINES MEDIKAMENTS ZUR BEHANDLUNG VON BRUST-, EIERSTOCK-, ENDOMETRIUM-KREBS, MYELOM UND MENINGIOM
UTILISATION DE 11BETA-(4-ACETYLPHENYL)-17BETA-HYDROXY-17ALPHA-(1,1,2,2-PENTAFLUOROETHYL)ESTRA-4,9-DIEN-3-ONE POUR LA PREPARATION D'UNE MEDICAMENT POUR LE TRAITEMENT DE CANCER DE SEIN, D'OVAIRE, DE L'UTERUS, DU MYELOME ET DU MENINGIOME

(30) Priority: 18.10.2000 EP 00250342; 18.10.2000 US 240991 P
(43) Date of publication of application: 16.07.2003
(73) Proprietor: Schering Aktiengesellschaft, 13353 Berlin (DE)
(72) Inventor: HOFFMANN, Jens, 16567 Mühlenbeck (DE); LICHTNER, Rosemarie, 10823 Berlin (DE); SIEMEISTER, Gerd, 13503 Berlin (DE); SCHNEIDER, Martin, 13469 Berlin (DE); FUHRMANN, Ulrike, 10587 Berlin (DE)
(86) International application number: PCT/EP2001/012006
(87) International publication number: WO 2002/032432

(56) References cited:
- DE-A- 19 706 061

## Description

### Field of the Invention

The present invention relates to the use of the antiprogestin 11β-(4-acetylphenyl)-17β-hydroxy-17α-(1,1,2,2,2-pentafluoroethyl)-estra-4,9-dien-3-one or a pharmaceutically acceptable derivative or analogue thereof for the preparation of a medicament for the treatment of a type of cancer selected from the group of breast cancer, ovarian cancer, endometrial cancer, myeloma and meningioma, wherein an indicator of high risk is an increased amount of tumor cells in the S-phase of the cell cycle.

### Background of the Invention

Antiprogestins represent a relatively new and promising class of therapeutic agents that could have significant impact on the treatment of hormone-dependent tumors and other diseases. Although antiprogestins were originally created with regard to medicinal non-surgical termination of pregnancy, certain antiprogestins have gained considerable importance, e.g., in the endocrine therapy of those breast cancers which possess receptors for progesterone (T. Maudelonde et al., in: J.G.M. Klijn et al., *Hormonal Manipulation of Cancer: Peptides, Growth Factors and New (Anti) Steroidal Agents,* Raven Press, New York, 1987, pp. 55-59).

This new strategy in endocrine therapy is based on the antitumor activity of antiprogestins in progesterone receptor positive human breast cancer cell lines *in vitro* and in several hormone-dependent mammary tumors of the mouse and rat *in vivo.* In particular, the antitumor mechanism of the antiprogestins onapristone and mifepristone (RU 486) has already been investigated using the hormone-dependent MXT mammary tumor model of the mouse as well as the DMBA- and the NMU-induced mammary tumor models of the rat (M. R. Schneider et al., *Eur. J. Cancer Clin. Oncol.,* Vol. 25, No. 4, pp. 691-701, 1989; H. Michna et al., *Breast Cancer Research and Treatment* 14:275-288, 1989; H. Michna, *J. Steroid. Biochem.* Vol. 34, Nos 1-6, pp. 447-453, 1989). However, due to low activity and adverse side effects involved with e.g. mifepristone this compound could not be recommended as a single agent in the management of breast cancer (D. Perrault et al., *J. Clin. Oticol.* 1996 Oct, 14(10), pp.2709-2712). Furthermore, mifepristone exhibits strong antiglucocorticoid side effects (cf. L.M. Kettel et al., *Fertil. Steril.* 1991 Sep, 56(3), pp. 402-407; X. Bertagna, *Psychoneuroendocrinology* 1997; 22 Suppl. 1, pp. 51-55).

The determination of the percentage of tumor cells in the respective phases of the cell cycle can be performed by the powerful DNA flow cytometry method (cf. G. M. Clark et al., *N. Engl. J. Med.* 320,1989, March, pp.627-633; L. G. Dressler et al., *Cancer* 61(3), 1988, pp. 420-427 and literature cited therein). It has thus been shown that the stages of the cell cycle of a tumor cell, and specifically, the number of tumor cells in certain stages of the cycle, may be an important clinical predictor of disease progression and success of therapy. The number of cells in the S-phase of the cell cycle are particularly important in this regard.

EP 0 495 825 B I discloses the use of antiprogestins (competitive progesterone antagonists) for the production of medicaments for the treatment of mammary carcinomas having an increased content of tumor cells in the S-phase of the cell cycle, which is considered to be a high risk factor. This is based on the observation that antiprogestins are capable of blocking the progression of tumor cells in the G₀G₁-phase of the cell cycle resulting in a substantial decrease of tumor cells in the S-phase. This effect was however not observed with the standard breast cancer therapy tamoxifen, estrogen therapy or ovariectomy. The antiprogestins tested in EP 0 495 825 B1 are 11β-[4-N,N-dimethylamino)-phenyl]-17α-hydroxy-17β-(3-hydroxypropyl)-13α-methyl-4,9(10)-gonadien-3-one and 11β-(4-acetylphenyl)-17β-hydroxy-17α-(prop-1-inyl)-4,9(10)-estradien-3-one.

17a-fluoroalkylsteroids having strong antiprogestin activity as well as methods for producing them are described in WO 98/34947. WO 98/34947 does not discuss or investigate the role that the 17a-fluoroalkylsteroids disclosed therein may play in cell apoptosis or cell cycle arrest.

Given the potential value of agents that induce apoptosis in cells, e.g., in the case of tumor cells, by blocking progression in the G₀G₁-phase, it is desirable to identify further agents, e.g., antiprogestins, having this specific mechanism of action. Such agents would have potential application in treating and preventing certain types of cancer, such as breast cancer, wherein an indicator of high risk is an increased amount of tumor cells in the S-phase of the cell cycle.

### Object of the Invention

It is thus an object of the present invention to further investigate the mode of action of antiprogestins in inhibiting hormone-dependent diseases such as breast cancer and to provide a method for the targeted induction of apoptosis in cells.

Surprisingly, the inventors have discovered that the antiprogestin 11β-(4-acetylphenyl)-17β-hydroxy-17α-(1,1,2,2,2-pentafluoroethyl)-estra-4,9-dien-3-one (or a pharmaceutically acceptable derivative or analogue thereof) may be used for the induction of apoptosis in a cell.

### Summary of the Invention

The present invention is based on the unexpected observation that the antiprogestin 1 Ip-(4-acetylphenyl)-17β-hydroxy-17α-(1,1,2,2,2-pentatluoroethyl)-estra-4,9-dien-3-one (hereinafter referred to as "antiprogestin (I)") induces apoptosis and cell death in the tumor cells of standard breast cancer tumor models. It was found that antiprogestin (I) is capable of inducing apoptosis in cells via the initiation of terminal differentiation.

Thus, the present invention provides the use of antiprogestin (I) or a pharmaceutically acceptable derivative or analogue thereof for the preparation of a medicament for the treatment of breast cancer, ovarion cancer, endometrial cancer, myclona and meningoma. Preferably, the induction of apoptosis is caused by the initiation of terminal differentiation. The cell is preferably a mammalian cell, more preferably a human cell and most preferably a tumor cell, wherein the tumor is preferably breast cancer.

Another aspect of the present invention is the use of antiprogestin (I) or a pharmaceutically acceptable derivative or analogue thereof for the preparation of a medicament for the treatment of types of cancer wherein an indicator of high risk is an increased amount of tumor cells in the S-phase of the cell cycle.

A further aspect of the present invention is the use of antiprogestin (I) or a pharmaceutically acceptable derivative or analogue thereof for the induction of apoptosis in a cell *in vitro.* Preferably, the cell is a mammalian cell, more preferably a human cell and most preferably a tumor cell, wherein the tumor is preferably breast cancer.

Another aspect of the present invention is a method of inducing apoptosis in a cell by administering an effective amount of antiprogestin (I) to the cell. This method may be applied *in vitro* or *in vivo.* Preferably, the cell is a mammalian cell, more preferably a human cell and most preferably a tumor cell, wherein the tumor is preferably breast cancer.

Due to the ability to induce cell apoptosis the antiprogestin (I) or a pharmaceutically acceptable derivative or analogue thereof may be used for the treatment of certain types of cancer, such as breast cancer, wherein an indicator of high risk is an increased amount of tumor cells in the S-phase of the cell cycle. Other types of cancer or hormone-dependent diseases that may be affected and treated by antiprogestin (I) due to its ability to induce cell apoptosis may include, e.g., breast cancer, ovarian cancer, endometrial cancer, myeloma, meningoma, i.e. diseases which substantially originate or are influenced by the presence of hormone receptors and/or hormone-dependent pathways.

### Brief Description of the Figures

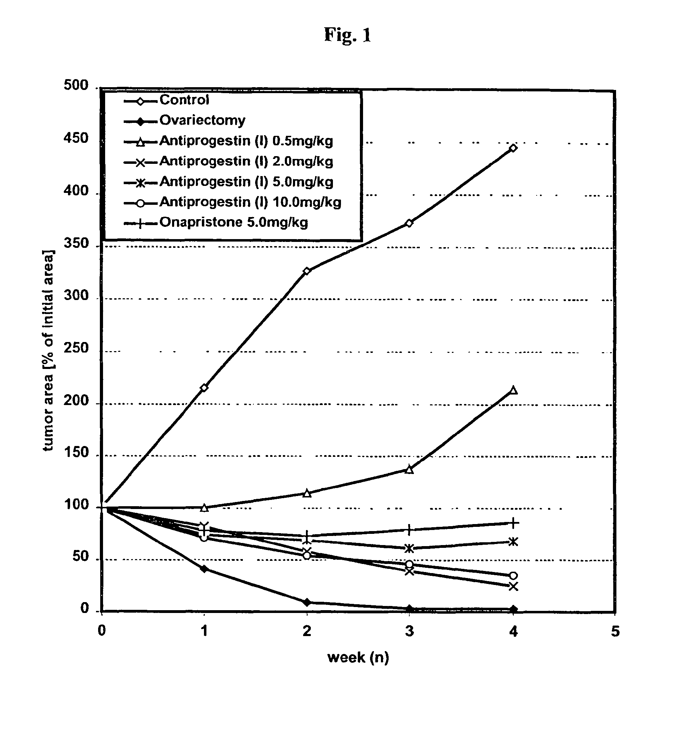

Figure 1 shows the tumor growth inhibiting effect as a result of the induction of apoptosis by antiprogestin (I) in a dose-response study in the DMBA-induced mammary carcinoma of the rat, compared with a control, the antiprogestin onapristone as well as ovariectomy. The study was performed with 0.5, 2.0, 5.0 and 10.0 mg/kg s.c. daily doses of antiprogestin (I).

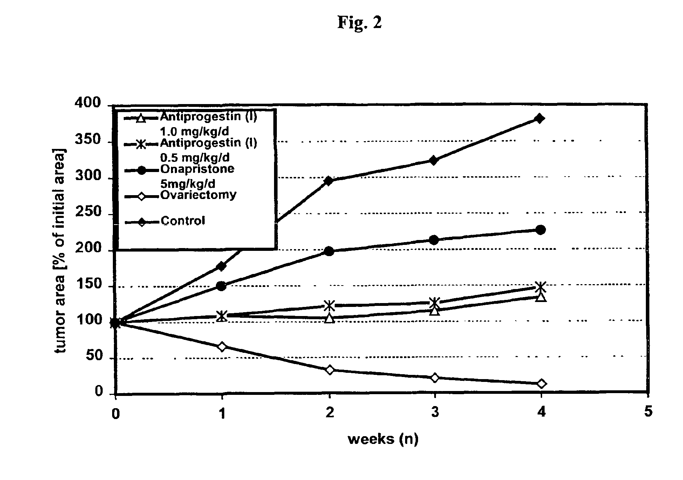

Figure 2 shows the tumor growth inhibiting effect as a result of the induction of apoptosis by antiprogestin (I) in the NMU-induced mammary carcinoma of the rat, compared with a control and ovariectomy. The study was performed with 0.5 and 1.0 mg/kg s.c. daily doses of antiprogestin (I).

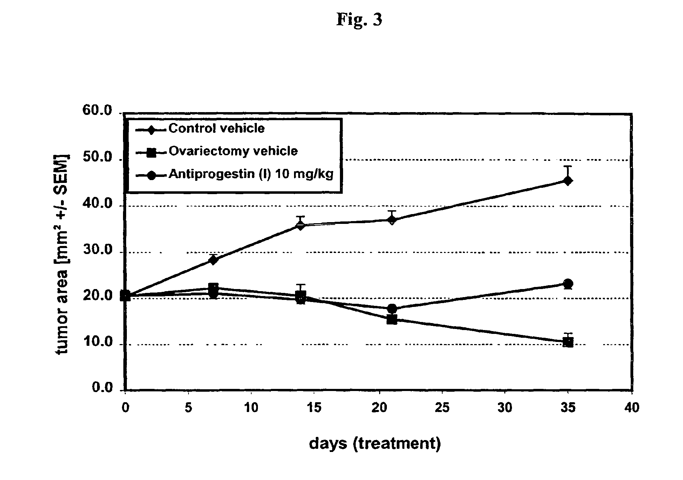

Figure 3 shows the induction of apoptosis and thus the tumor growth inhibiting effect of antiprogestin (I) in a 10 mg/kg s.c. dose on xenotransplanted human T47D tumors in scid mice, compared to a control and ovariectomy.

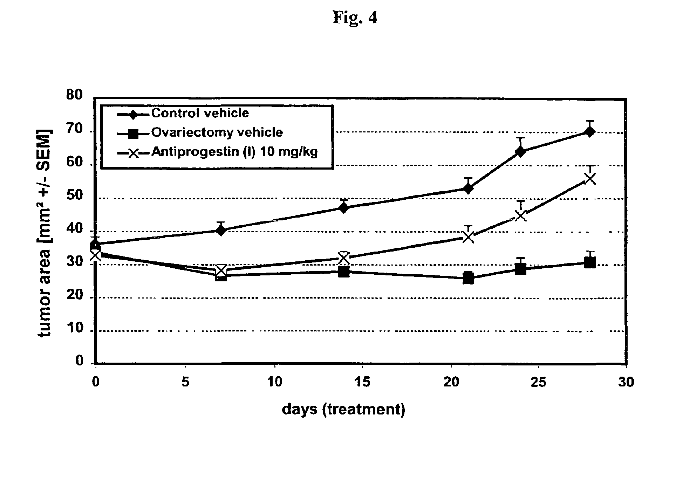

Figure 4 demonstrates the induction of apoptosis and thus the tumor growth inhibiting effect of a 10 mg/kg s.c. dose of antiprogestin (I) in the MCF-7 human breast cancer model in scid mice, compared to a control and ovariectomy.

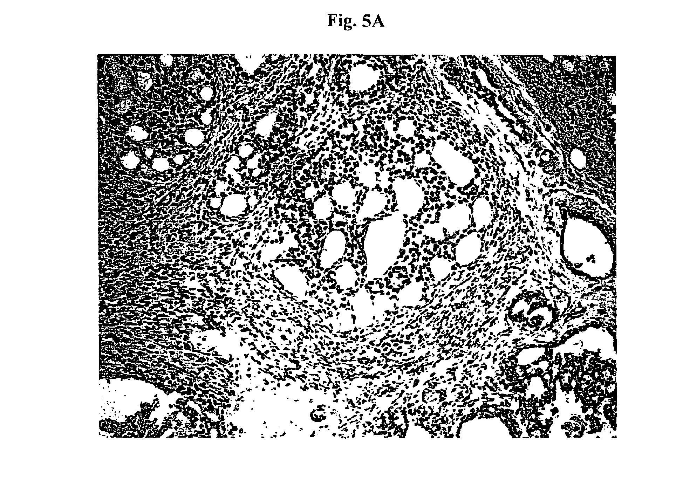

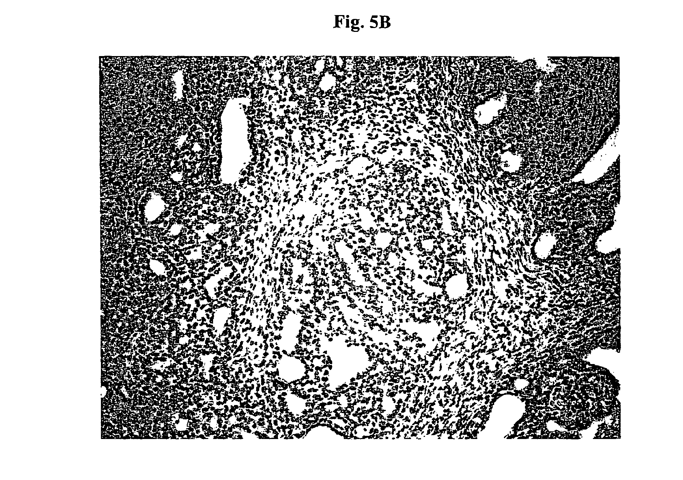

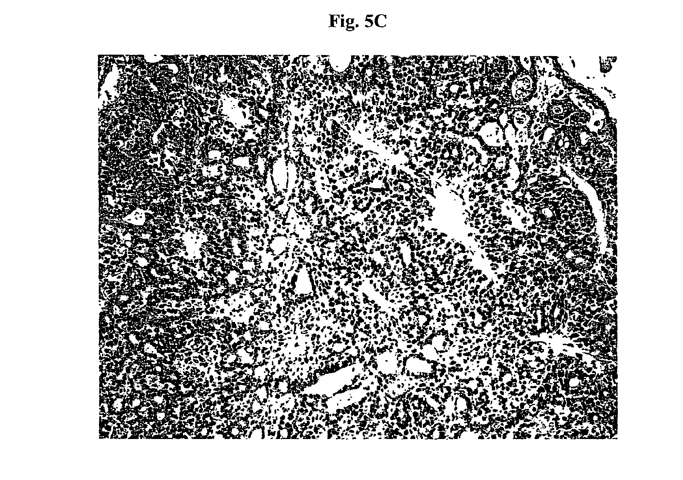

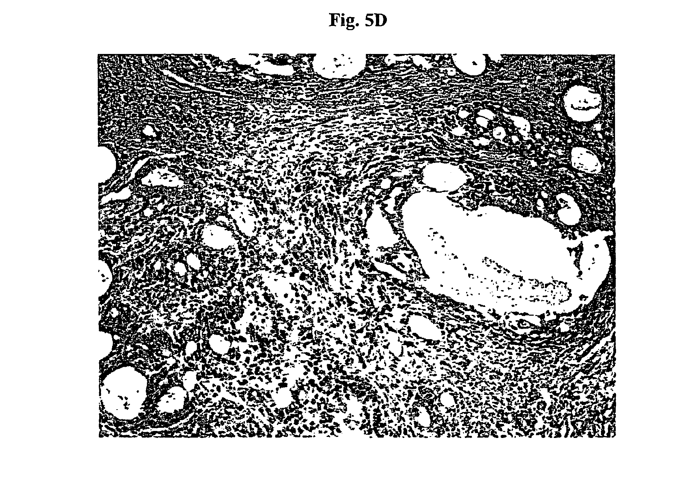

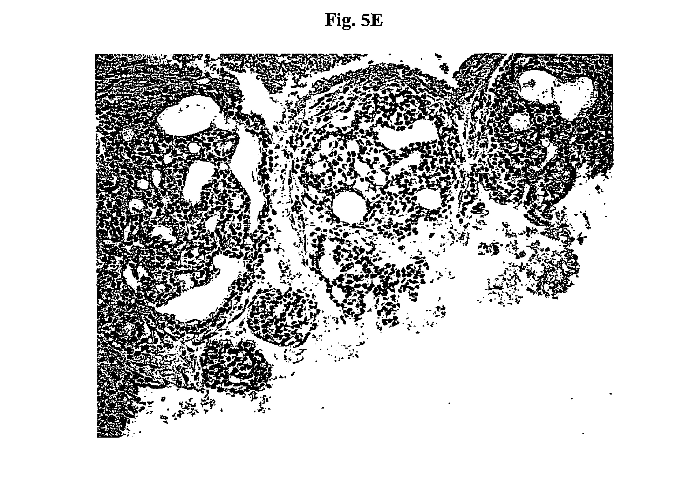

Figures 5A to 5F show histological data relating to the induction of apoptosis in the NMU-induced breast cancer model in rat (cf. Example 5). In particular, figure 5A shows that tumors treated with antiprogestin (I) display ductal and acinous formations, usually filled with secretory material, compared to the control (figure 5B). Figure 5C shows untreated NMU-induced breast cancer tissue with high PCNA (proliferating cell nuclear antigen) immunoreactivity as compared to NMU-induced breast cancer tissue treated with antiprogestin (I) (figure 5D), which exhibits low PCNA immunoreactivity. Figure 5E shows the appearance of apoptosis in antiprogestin (I)-treated NMU-induced breast cancer tissue, compared to the control (figure 5E).

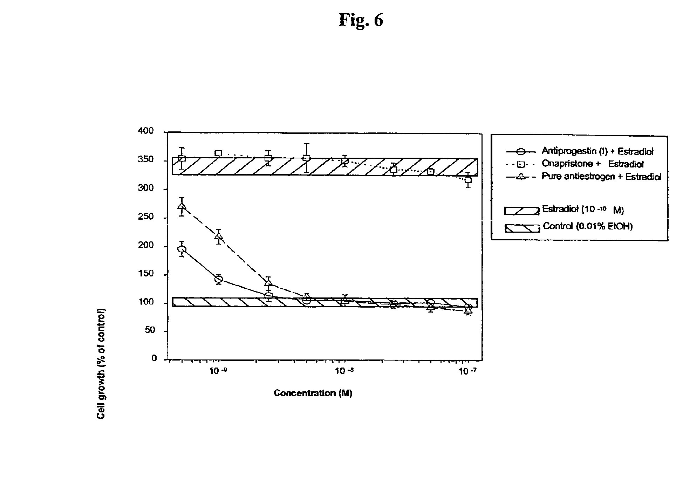

Figure 6 demonstrates the tumor growth inhibiting effect of antiprogestin (I) in the T47D breast cancer cell line (stimulated by estradiol) with an effective threshold concentration of 10⁻⁹ to 10⁻⁸ mol/l, compared with the antiprogestin onapristone and the pure antiestrogen 11β-fluoro-7α-{5-[N-methyl-N-3-(4,4,5,5,5-pentafluoropentylthio)-propylamino]-pentyl}-estra-1,3,5(10)-trien-3,17β-diol (WO 98/07740).

### Detailed Description of the Invention

Antiprogestin (I) ― 11 β-(4-acetylphenyl)-17β-hydroxy-17α-(1, 1,2,2,2-pentafluoroethyl)-estra-4,9-dien-3-one ― is represented below by formula (I):

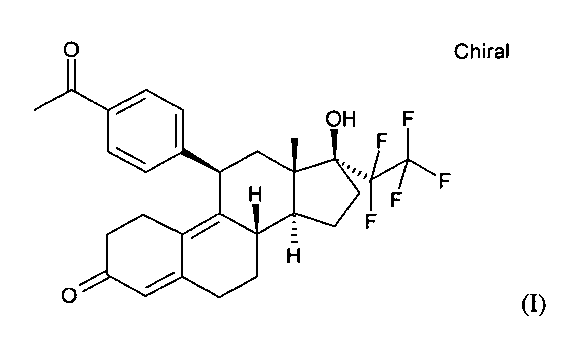

Antiprogestin (I) (or a pharmaceutically acceptable derivative or analogue thereof) is a valuable pharmaceutical agent having strong antiprogestin activity. Antiprogestin (I) can be used according to the present invention for the induction of apoptosis in cells.

However, it should also emcompass compounds capable of inhibiting the biosynthesis of progestins.

Pharmaceutically acceptable derivatives or analogues of antiprogestin (I) in the context of the present invention may include, for example, any one of the inventive compounds disclosed in WO 98/34947.

The studies performed in the context of the present invention show the potent tumor-inhibiting properties of the antiprogestin (I) in a variety of hormone-dependent tumor models (see Examples 1 to 6). It is further demonstrated that the tumor inhibiting activity of antiprogestin (I) as a result of the induction of apoptosis is stronger than conventional anti-tumor agents, such as, the antiestrogen tamoxifen. The treatment of breast cancer using the antiprogestin (I) according to the present invention is even superior to ovariectomy.

Application of antiprogestin (I) in the various tumor models as demonstrated below in the Examples revealed an accumulation of tumor cells in the G₀G₁ phase of the cell cycle together with a significant and biologically relevant reduction in the number of cells in the S and G₂M phase of the cell cycle. These results indicate an induction of differentation. Differentiation-specific G₁ arrest has already been proposed earlier for other stem cell systems (see J.J. Wille Jr., *Cancer Res.* 1982, 42(12):5139-46; R.E. Scott, *J. Cell. Biol.* 1982, 94(2):400-405).

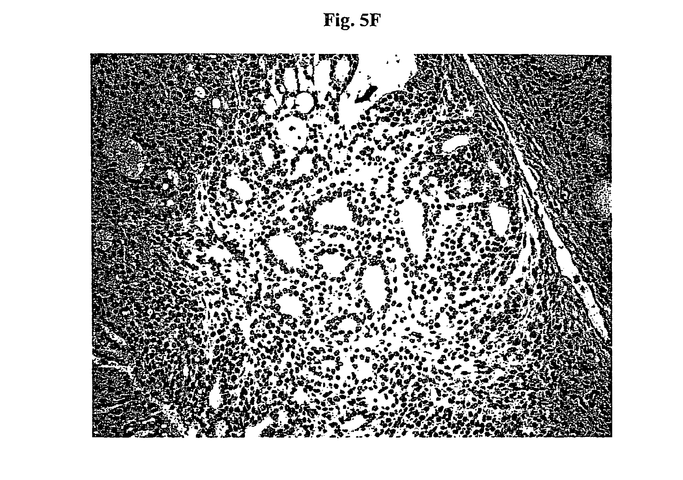

The experimental results obtained in the various tumor models revealed that treatment with antiprogestin (I) seems to trigger differentiation of the mitotically active polygonal tumor cells towards glandular structures and acini with a massive sequestering of secretory products, as well as towards spindle-shaped necrobiotic subpopulations (see Example 5 and in particular figures 5A and 5B). Whereas tumor size, mitotic index and the grade of malignancy decreased distinctly, the volume fraction of glandular structures in the tumors as well as the appearance of apoptosis increased 3-fold compared to the controls (see Example 5, figures 5E and 5F).

Without limitation to any theory, these results indicate that the main mechanism of the antitumor action of antiprogestin (I) in the tested models is a direct progesterone-receptor-mediated antiproliferative effect at the level of the tumor cells, via the induction of terminal differentiation associated with terminal cell death. In this manner, antiprogestin (I) appears to be capable of eliminating the intrinsic block in terminal differentiation inherent in malignant tumor cells in progesterone receptor-positive tumors. This antiproliferative effect of antiprogestin (I) seems to be dissociated from the antihormone (antiprogestional) activity of antiprogestin (I).

Agents such as antiprogestin (I) that induce apoptosis in cells, for example, in the case of tumor cells, by blocking progression in the G₀G₁-phase, have potential applications for treating and preventing numerous conditions. Such agents, including antiprogestin (I), may be used for treating those cancers where an indicator of high risk is an increased amount of tumor cells in the S-phase of the cell cycle, such as in breast cancer.

Thus one aspect of the present invention is the use of antiprogestin (I) or a pharmaceutically acceptable derivative or analogue thereof for preparation of a medicament for the induction of apoptosis in a cell. In a preferred embodiment, the use of antiprogestin (I) or a pharmaceutically acceptable derivative or analogue thereof relates to a medicament for the induction of apoptosis in a tumor cell, preferably a breast tumor cell, in a human. Such medicament could be beneficial in the treatment of hormone-dependent diseases such as breast cancer, wherein an indicator of high risk is an increased amount of tumor cells in the S-phase of the cell cycle.

The manufacture of the medicaments may be performed according to methods known in the art. Commonly known and used adjuvants as well as further suitable carriers or diluents may be used. Suitable carriers and adjuvants may be such as recommended for *pharmacy, cosmetics and related fields in: Ullmann's Encyclopedia of Technical Chemistry,* Vol. 4, (1953), pp. 1-39; *Journal of Pharmaceutical Sciences,* Vol. 52 (1963), p. 918ff; H.v.Czetsch-Lindenwald, "Hilfsstoffe Wr Pharmazie und angrenzende Gebiete"; *Pharm. Ind.* 2, 1961, p.72ff; Dr. H.P. Fiedler, *Lexikon der Hilfsstoffe für Pharmazie, Kosmetik und angrenzende Gebiete,* Cantor KG, Aulendorf in Württemberg, 1971.

Antiprogestins suitable for the purposes of the present invention, preferably antiprogestin (I) or a pharmaceutically acceptable derivative or analogue thereof, can be incorporated into pharmaceutical compositions according to known methods of preparing galenics for oral or parenteral, e.g., intraperitoneal, intramuscular, subcutaneous or percutaneous application. They can also be implanted into tissue. Implants can comprise as inert materials e.g. biologically degradable polymers or synthetic silicones such as e.g. silicone rubber.

They can be administered in the form of tablets, pills, dragees, gel capsules, granules, suppositories, implants, injectable sterile aqueous or oily solutions, suspensions or emulsions, ointments, creams, gels or by intravaginal (e.g., vaginal rings) or intrauterine systems (e.g., diaphragms, loops).

For the preparation of a medicament for oral administration, the antiprogestins suitable for the purposes of the present invention as defined above can be admixed with commonly known and used adjuvants and carriers such as for example, gum arabic, talcum, starch, sugars such as, e.g., mannitose, methyl cellulose, lactose, gelatin, surface-active agents, magnesium stearate, aqueous or non-aqueous excipients, paraffin derivatives, crosslinking agents, dispersants, emulsifiers, lubricants, conserving agents and flavoring agents (e.g., ethereal oils). In a pharmaceutical composition, the antiprogestin may be dispersed in a microparticle, e.g. a nanoparticulate, composition.

In order to further enhance the bioavailability of the active agent, the antiprogestins suitable for the purposes of the present invention as defined above can also be formulated as cyclodextrin clathrates by reacting them with α-, β- or γ-cyclodextrines or derivatives thereof according to the method as disclosed in PCT/EP95/02656.
For parenteral administration the antiprogestins suitable for the purposes of the present invention as defined above can be dissolved or suspended in a physiologically acceptable diluent, such as, e.g., oils with or without solubilizers, surface-active agents, dispersants or emulsifiers. As oils for example and without limitation, olive oil, peanut oil, cottonseed oil, soybean oil, castor oil and sesame oil may be used.

The amount to be administered (i.e., a "pharmaceutically effective amount") varies within a broad range and depends on the condition to be treated and the mode of administration. It can cover any amount efficient for the intended treatment. Determining a "pharmaceutically effective amount" is within the purview of the person skilled in the art.

One unit dose may represent about 0.1 to 100 mg active agent(s). For administration to humans, the daily dose of the active agent(s) is about 0.1 to 400 mg, preferably 10 to 100 mg, most preferably 50 mg.

The medicaments can also be administered via a depot injection or an implant preparation, optionally for sustained delivery of the active agent(s).

The preferred mode of administration is oral administration. The antiprogestins for use according to the invention, and in particular, antiprogestin (I) are particularly suitable for oral administration.

According to all aspects of the present invention it is also possible to combine at least one antiprogestin as defined above, in particular antiprogestin (I) or a pharmaceutically acceptable derivative or analogue thereof, with at least one antiestrogen, because many hormone-dependent diseases, in particular breast cancer, exhibit not only progesterone receptors, but also estrogen receptors. The antiestrogen may be administered either simultaneously with or sequentially to the antiprogestin, and in particular with/to antiprogestin (I) or a pharmaceutically acceptable derivative or analogue thereof. The amount of antiprogestin and antiestrogen may be equal or one component may be more predominant than the other, such as in an antiprogestin:antiestrogen ratio of 1:50 to 50:1, preferably 1:30 to 30:1, and most preferably 1:15 to 15:1.

Examples of suitable antiestrogens for use according to the invention are non-steroidal antiestrogens, such as tamoxifen and nafoxidine as well as raloxifen, faslodex and EM800. Examples of steroidal antiestrogens include those disclosed in EP 0 348 341 A and those disclosed in WO 98/07740, in particular, 11β-flouro-7α-{5-[N-methyl-N-3-(4,4,5,5,5-pentaflouropentylthio-propylamino]-pentyl}-estra-1,3,5(10)-trien-3,17β-diol, or those disclosed in WO 99/33855, in particular 11β-flouro-7α-{5-[methyl-(7,7,8,8,9,9,10,10,10-nonafluoro-decyl)-amino]-pentyl} -estra-1,3,5(10)-trien-3,17β-diol or pharmaceutically acceptable derivatives or analogues thereof. Aromatase inhibitors having an antiestrogen effect, such as those disclosed on pages 7 to 8 of EP 0 495 825 B1 may also be used as antiestrogens.

Another aspect of the present invention is the use of antiprogestin (I) or a pharmaceutically acceptable derivative or analogue thereof for the preparation of a medicament for the treatment of a type of cancer wherein an indicator of high risk is an increased amount of tumor cells in the S-phase of the cell cycle. The number of tumor cells in the S-phase may be determined by DNA flow cytometry as described in Dressler et al., "DNA Flow Cytometry and Prognostic Factors in 1331 Frozen Breast Cancer Specimens," *Cancer,* Vol. 61(3), 1988, pp. 420-427; see also McGuire & Dressler, "Emerging Impact of Flow Cytometry in Predicting Recurrence and Survival in Breast Cancer Patients," *JNCI,* Vol. 75(3), 1985, pp. 405-409. A high risk amount of tumor cells in the S-phase indicates a particularly suitable candidate for the use according to the invention. In the case of antiprogestin (I), the advantage arises from both the potent anti-tumor effect, as evidenced by the standard animal models (see Examples 1 to 4), and the mechanism of action of this agent of inducing apoptosis (see in particular Example 5) and cell cycle arrest.

In an alternative aspect the present invention provides a method for inducing apoptosis in a cell. The cell is preferably a mammalian cell and most preferably a human cell, and the method may be applied *in vitro* or *in vivo.* Preferably, apoptosis is induced via the mechanism of initiating terminal differentiation, for example, by the administration of antiprogestin (I) or a pharmaceutically acceptable derivative or analogue thereof. In the method, an effective amount of antiprogestin (I) or a pharmaceutically acceptable derivative or analogue thereof may be applied to the cells in question. For example in the T47D breast cancer cell line, whose growth is stimulated by the administration of estradiol, antiprogestin (I) induced a complete inhibition of cell growth with an effective threshold concentration of between 10⁻⁹ and 10⁻⁸ mol (see Example 6 and figure 6). This is especially surprising as the known antiprogestin onapristone has no reducing effect on cell growth in this tumor model. Thus, antiprogestin (I) is superior with regard to potency and efficacy to other antiprogestins such as onapristone and to antiestrogens such as tamoxifen and even to pure antiestrogens such as 11β-fluoro-7α-{5-[N-methyl-N-3-(4,4,5,5 ,5-pentafluoropentylthio)-propylamino]-pentyl}-estra-1,3,5(10)-trien-3,17β-diol (WO 98/07740).

The role of antiprogestin (I) in the induction of apoptosis in the cell indicates that this antiprogestin (or a pharmaceutically acceptable derivative or analogue thereof) may be useful in a host of conditions, particularly hormone-dependent conditions, where induction of apoptosis is particularly desired. Specifically, it may be used in the treatment of such diseases as breast cancer, ovarian cancer, endometrial cancer, myeloma, anowlatory infertility, meningoma, i.e., diseases which substantially originate or are influenced by the presence of hormone receptors and/or hormone-dependent pathways. Antiprogestins, such as antiprogestin (I), may thus be further used for the preparation of medicaments for inducing apoptosis or cell death for the treatment of hormone-dependent diseases as already described above.

The invention is further illustrated in the examples. The following examples are not to be understood as a limitation.

### Examples

### Example 1:

### Dose-response study in the DMBA-induced tumor model

### Materials and Methods:

Immature female Sprague-Dawley rats (49 - 51 days old; 10 animals/group) were used in this study. Mammary tumors were induced by a single oral administration of 10 mg 7,12-dimethylbenz[a]anthracene (DMBA, Serva/Heidelberg). Rats with at least one established tumor with a size of more than 150 mm² were treated for 4 weeks by: 1) solvent control, 2) ovariectomy at treatment start, 3) antiprogestin (I), 0,5 mg/kg s.c., 4) antiprogestin (I), 2 mg/kg s.c., 5) antiprogestin (1), 5 mg/kg s.c., 6) antiprogestin (I), 10 mg/kg s.c., and 7) onapristone, 5 mg/kg, s.c., daily. As a parameter for growth inhibition the change of tumor area (in % with respect to initial tumor size) determined by weekly caliper measurements was used. For statistical analysis of intergroup differences of mean values the Kruskal-Wallis-test was used. For a further description and evaluation of the DMBA prevention model, see R.G. Metha, *European Journal of Cancer* 36 (2000), pp. 1275-1282.

### Results:

In intact control animals, progressive tumor growth was observed, whereas ovariectomy caused a considerable tumor regression in 90% of the animals. Treatment with antiprogestin (I) at doses of or above 2 mg/kg resulted in a significant induction of apoptosis resulting in inhibition of tumor growth compared with the control (see fig. 2). There was a clear dose-response relationship. Whereas treatment with 0.5 mg/kg antiprogestin (I) did not significantly prevent the tumor from growing, at 2 mg/kg maximal induction of apoptosis and thus growth inhibition was observed. In this group a complete tumor regression was seen in 50% of the rats. The effect of the highest dose of antiprogestin (I) tested in this experiment (10 mg/kg), was comparable to that of 2 mg/kg. Onapristone (5 mg/kg, s.c.) was distinctly less effective than antiprogestin (I) at comparable doses.

### Conclusion:

In the DMBA-induced mammary tumor model in the rat, antiprogestin (I) strongly induced apoptosis in the tumor cells and thus completely suppressed the tumor growth in intact animals. It was found that 2 mg/kg antiprogestin (I) has a maximal apoptotic effect on tumor cells. Antiprogestin (I) was distinctly superior to onapristone regarding the inhibition of tumor growth.

### Example 2:

### Tumor growth inhibition in NMU-induced breast cancer model in rat

### Materials and Methods:

Tumors were induced by a single intravenous injection of NMU (nitrosomethylurea, 50 mg/kg) in female Sprague-Dawley rats (obtained from Tierzucht Schönwalde, age 50-55 days). Starting 10 days later, rats with at least one established tumor were treated for 4 weeks by: 1) solvent control, 2) ovariectomy at treatment start, 3) antiprogestin (I), 1.0 mg/kg/day, 4) antiprogestin (1), 0.5 mg/kg/day and 5) onapristone, 5 mg/kg/day. As a parameter for growth inhibition the change of tumor area (in % of initial tumor size) determined by weekly caliper measurements was used. For statistical analysis of intergroup differences of mean values the Kruskal-Wallis-test was used.

### Results:

In intact control animals, progressive tumor growth was observed, whereas ovariectomy caused a complete tumor growth inhibition. Treatment with antiprogestin (I) at doses of 0.5 or 1.0 mg/kg resulted in a significant inhibition of tumor growth due to the induction of apoptosis compared with the control (see fig. 2). Onapristone (5 mgi7cg) was distinctly less effective than antiprogestin (I) at the much lower dose of 0.5 mg/kg.

### Conclusions:

In the MNU-induced mammary tumor model in the rat, due to its potent ability to induce apoptosis in tumor cells, antiprogestin (I) completely suppresses the tumor growth in intact animals. Both doses (1.0 mg/kg as well as 0.5 mg/kg) of antiprogestin (I) have a significant apoptotic effect on tumor cells.

### Example 3:

### Human T47D breast cancer xenograft in scid mice

### Materials and Methods:

Female Fox Chase scid mice (M&B) were supplemented with estradiol pellets (Innovative Research of America). T47D breast cancer cells, obtained from cell culture and suspended in matrigel, were implanted s.c. in the inguinal region of the mice. Treatment was started when the tumors were approximately 25 mm² in size. Treatment was continued until progression of the tumors. Experimental groups were: 1) control (vehicle), 2) ovariectomy, 3) antiprogestin (I), 10 mg/kg s.c. Tumor area was determined by caliper measurements. The Kruskal Wallis test was used for statistical analysis of intergroup differences of mean values.

### Results:

In the T47D breast cancer model, ovariectomy resulted in a considerable inhibition of tumor growth, compared with the rapid growth in the control. Fig. 3 clearly shows that the s.c. application of 10 mg/kg antiprogestin (I) induces apoptosis in the tumor cells. The effect of antiprogestin (1) is almost comparable to the effect of conventional estrogen deprivation therapy (ovariectomy).

### Conclusion:

The effect of antiprogestin (I) in inducing apoptosis and thus inhibiting the growth of the human T47D breast cancer xenografted in Fox Chase scid mice is comparable to the effect of standard estrogen deprivation therapy (ovariectomy) which is considered to be the maximum effective method of inhibiting growth of breast cancer in this model.

### Example 4:

### Human MCF-7 breast cancer xenograft in scid mice

### Materials and Methods:

Female Fox Chase scid mice (M&B) were supplemented with estradiol pellets (Innovative Research of America). MCF7 breast cancer cells, obtained from cell culture and suspended in matrigel, were implanted s.c. in the inguinal region of the mice. Treatment was started when the tumors were approximately 25 mm² in size. Treatment was continued until progression of the tumors. Experimental groups were: 1) control (vehicle), 2) ovariectomy, 3) antiprogestin (I), 10 mg/kg s.c. Tumor area was determined by caliper measurements. The Kruskal Wallis test was used for statistical analysis of intergroup differences of mean values.

### Results:

In the MCF7 breast cancer model, ovariectomy resulted in a considerable inhibition of tumor growth, compared with the rapid growth in the control. Fig. 4 clearly shows that the s.c. application of 10 mg/kg antiprogestin (I) induced apoptosis in the tumor cells. The effect of antiprogestin (1) is comparable to the effect of conventional estrogen deprivation therapy (ovariectomy).

### Conclusion:

The effect of antiprogestin (I) in inducing apoptosis and thus inhibiting the growth of the human MCF7 breast cancer xenografted in Fox Chase scid mice is comparable to the effect of standard estrogen deprivation therapy (ovariectomy).

### Example 5:

### NMU-induced breast cancer in rat (histology, proliferation index and TUNEL assay)

### Materials and Methods:

Tumors were induced by a single intravenous injection ofNMU (nitrosomethylurea, 50 mg/kg) in female Sprague-Dawley rats (obtained from Tierzucht Schönwalde, age 50-55 days). Rats with at least one established tumor with a size of more than 150 mm² were treated for 7 days by: 1) solvent control, 2) ovariectomy at treatment start, 3) antiprogestin (I), 3 mg/kg s.c., daily. At the end of treatment tumors were excised, fixed in formalin and embedded in paraffin. Histology, proliferation index and apoptosis induction assays were performed on these resected tumors.

*Histology:* For histology tissue slides were stained with haematoxilin and analyzed by microscopy.

*Proliferation Index:* To determine the proliferation index the expression of PCNA was determined. Proliferating cell nuclear antigen (PCNA) is a 36 kD nuclear protein associated with the cell cycle. Nuclear PCNA immunoreactivity is found in the proliferative compartment of normal tissues. A monoclonal antibody, that recognizes a fixation and processing resistant epitope has been used to investigate its tissue distribution.

*TUNEL (Apoptosis Test):* The biochemical hallmark of apoptosis is the degradation of the genomic DNA, an irreversible event that results in cell death. This characteristic DNA fragmentation is the result of the activation of nuclear endonucleases, which selectively cleave DNA at sites located between nucleosomal units. These DNA strand breaks were detected by enzymatic labeling of the 3'-OH termini with fluorescein-dUTP using terminal deoxynucleotidyl transferase (TUNEL, Terminal Deoxynucleotidyl Transferase-Mediated dUTP Nick End Labeling, cf. Gavrieli et al., J. Cell. Biol. 119, 493, 1992). Incorporated fluorescein was detected using the anti-fluorescein antibody alcaline phosphatase conjugate followed by alcaline phosphatase substrate reaction.

### Results:

*Histology:* After treatment with antiprogestin (I), the tissue sections from the NMU tumors displayed dysplastic ductal and acinous formations, usually filled with secretory material (Figure 5A). Moreover, the volume fraction of glandular structures in the tumors increased compared to controls (Figure 5B). In addition, the mammary tumors of antiprogestin (I) treated animals showed the morphological features of differentiation.

*Proliferation Index:* PCNA immunoreactivity is high in untreated NMU-induced breast cancer tissue (Figure 5C: Untreated control). The number of cells with PCNA immunoreactivity is reduced by induction of differentiation in NMU-induced breast cancer tissue from rats treated with antiprogestin (I) (Figure 5D). These data demonstrate that in breast cancer, treatment with antiprogestin reduces the proliferation index by induction of differentiation.

*TUNEL (Apoptosis):* Figure 5E demonstrates the appearance of apoptosis induced by antiprogestin (1) in NMU-induced breast cancer tissue in comparison with untreated control (Figure 5F). It is clearly evident that antiprogestin (I) alone was capable of inducing apoptosis in the NMU-induced breast cancer tissue and thus inhibited the growth of these tumors.

### Example 6:

### Antiproliferative activity of antiprogestin (I) in vitro in the T47D cell line

### Materials and Methods:

T47D cells were grown in charcoal-treated serum supplemented with 0.1 nM E2 (estradiol) plus antiprogestin (I) for 6 days with one medium change. Following fixation and subsequent staining with crystal violet, the absorbance was recorded and values normalized to the absorbance of untreated controls as described in R.B. Lichtner, *J*. *Steroid Biochem. Mol. Biol.* 1999, 71;181-189. The TUNEL assay is performed analogous to above Example 5 with the only difference that instead of tissue sections cells that are cultivated on microscopic slides are used for the assay.

### Results:

In this T47D cell line *in vitro* test, antiprogestin (I) exhibited potent tumor growth inhibiting activity with an effective threshold concentration as low as 10⁻⁹ to 10⁻⁸ mol/l whereas the antiprogestin onapristone did not show any inhibiting effect. Even the pure antiestrogen 11β-fluoro-7α-{5-[N-methyl-N-3-(4,4,5,5,5-pentafluoropentylthio)-propylamino]-pentyl}-estra-1,3,5(10)-trien-3,17β-diol (WO 98/07740) was distinctly less effective than antiprogestin (I) (see figure 6).

### Conclusion:

Antiprogestin (I) according to the present invention induces complete inhibition of estradiol-stimulated T47D cell growth at very low concentrations and is thus superior regarding potency and efficacy to other antiprogestins tested such as onapristone and to the pure antiestrogen 11β-fluoro-7α-{5-[N-methyl-N-3-(4,4,5,5,5-pentafluoropentylthio)-propylamino]-pentyl}-estra-1,3,5(10)-trien-3,17β-diol.

## Claims

1. Use of the antiprogestin 11 B-(4-acetylphenyl)-17G-hydroxy-17a.-(1,',2,2,2-pentafluoroethyl)estra-4,9-dien-3-one or a pharmaceutically acceptable derivative or analogue thereof for the preparation of a medicament for the treatment of a type of cancer selected from the group of breast cancer, ovarian cancer, endometrial cancer, myeloma and meningioma with a high risk amound of tumor cells in the S-phase, as a result of the induction of apoptosis by applying said antiprogestin or pharmaceutically acceptable derivative or analogue thereof in a daily dose of 0.1 to 400 mg/kg.

2. Use according to claim 1 wherein the cancer is breast cancer.

## Patentansprüche

1. Verwendung des Antigestagens 11β-(4-Acetylphenyl)-17β-hydroxy-17α-(1,1,2,2,2-pentafluoroethyl)-östra-4,9-dien-3-on oder eine pharmazeutisch annehmbaren Derivats oder Analogons davon zur Herstellung eines Medikaments zur Behandlung einer Krebsart, ausgewählt aus der Brustkrebs, Ovarialkrebs, Endometriumkrebs, Myelom und Meningiom umfassenden Gruppe, mit einer Anzahl Tumorzellen in der S-Phase, die ein hohes Risiko darstellt, durch Induktion von Apoptose durch Verabreichung dieses Antigestagens oder eines pharmazeutisch annehmbaren Derivats oder Analogons davon in einer Tagesdosis von 0,1 bis 400 mg/kg.

2. Verwendung nach Anspruch 1, worin der Krebs Brustkrebs ist.

## Revendications

1. Utilisation de l'antiprogestine 11β-(4-acétylphényl) -17Q-hydroxy-17a- (1,1,2,2,2-pentafluoroéthyl)estra-4,9-diène-3-one ou d'un dérivé ou analogue de celle-ci acceptable d'un point de vue pharmaceutique pour la préparation d'un médicament destiné au traitement d'un type de cancer choisi dans le groupe des : cancer du sein ; cancer de l'ovaire ; cancer de l'endomètre ; myélome ; et méningiome, avec une quantité, à risque élevé, de cellules tumorales dans la phase S comme résultat de l'induction d'une apoptose par une application de ladite antiprogestine ou d'un dérivé ou analogue de celle-ci acceptable d'un point de vue pharmaceutique en une dose quotidienne de 0,1 à 400 mg/kg.

2. Utilisation selon la revendication 1, dans laquelle le cancer est un cancer du sein.
